# EUROPEAN PATENT SPECIFICATION

(11) **EP 1 793 874 B1**
(45) Date of publication and mention of the grant of the patent: **19.12.2012**
(21) Application number: 05781789.2
(22) Date of filing: 30.06.2005
(51) Int. Cl.: A61L 27/18

(54) **Soft tissue constructs comprising adult mesenchymal stem cells**
Weichgewebekonstrukte enthaltend adulte mesenchymale Stammzellen
Dispostif pour tissu souple comprenant des cellules souches mésenchymales adultes

(30) Priority: 30.06.2004 US 584657 P
(43) Date of publication of application: 13.06.2007
(73) Proprietor: THE BOARD OF TRUSTEES OF THE UNIVERSITY OF ILLINOIS, Urbana, IL 61801 (US)
(72) Inventor: MAO, Jeremy, Chicago, IL 60657 (US)
(74) Representative: Jappy, John William Graham
(86) International application number: PCT/US2005/023318
(87) International publication number: WO 2006/004951

(56) References cited:
- WO-A-01/82991
- WO-A-2005/025493
- WO-A-2005/033272
- ALHADLAQ A ET AL: "Tissue-engineered neogenesis of human-shaped mandibular condyle from rat mesenchymal stem cells." JOURNAL OF DENTAL RESEARCH, vol. 82, no. 12, December 2003 (2003-12), pages 951-956, XP002356383 ISSN: 0022-0345
- NUTTELMAN CHARLES R ET AL: "In vitro osteogenic differentiation of human mesenchymal stem cells photoencapsulated in PEG hydrogels." JOURNAL OF BIOMEDICAL MATERIALS RESEARCH, vol. 68A, no. 4, 15 March 2004 (2004-03-15), pages 773-782, XP002356384 ISSN: 0021-9304
- WILLIAMS CHRISTOPHER G ET AL: "In vitro chondrogenesis of bone marrow-derived mesenchymal stem cells in a photopolymerizing hydrogel." TISSUE ENGINEERING, vol. 9, no. 4, August 2003 (2003-08), pages 679-688, XP002356385 ISSN: 1076-3279
- SHIN HEUNGSOO ET AL: "In vitro cytotoxicity of unsaturated oligo(poly(ethylene glycol) fumarate) macromers and their cross-linked hydrogels." BIOMACROMOLECULES, vol. 4, no. 3, 27 February 2003 (2003-02-27), pages 552-560, XP002356386 ISSN: 1525-7797
- ALHADLAQ ADEL ET AL: "Engineered adipose tissue from human mesenchymal stem cells maintains predefined shape and dimension: Implications in soft tissue augmentation and reconstruction" TISSUE ENGINEERING, vol. 11, no. 3-4, March 2005 (2005-03), pages 556-566, XP002356387 ISSN: 1076-3279
- PATEL PARUL NATVAR ET AL: "Rheological and recovery properties of poly(ethylene glycol) diacrylate hydrogels and human adipose tissue" JOURNAL OF BIOMEDICAL MATERIALS RESEARCH, vol. 73A, no. 3, 15 April 2005 (2005-04-15), pages 313-319, XP002356388 ISSN: 1549-3296(print) 1552-4965(ele
- HONG LIU ET AL: "Ex vivo adipose tissue engineering by human marrow stromal cell seeded gelatin sponge" ANNALS OF BIOMEDICAL ENGINEERING, vol. 33, no. 4, April 2005 (2005-04), pages 511-517, XP002356389 ISSN: 0090-6964
- PITTENGER M F ET AL: "MULTILINEAGE POTENTIAL OF ADULT HUMAN MESENCHYMAL STEM CELLS" SCIENCE, AMERICAN ASSOCIATION FOR THE ADVANCEMENT OF SCIENCE,, US, vol. 284, no. 5411, 2 April 1999 (1999-04-02), pages 143-147, XP000867221 ISSN: 0036-8075

## Description

Soft tissue including adipose tissue and fibroblasts is biologically engineered to a predefined shape and dimensions using adult mesenchymal stem cells embedded within a biocompatible hydrogel matrix.

### BACKGROUND

Repair of soft tissue defects represent substantial challenges for contemporary medical practice. Many types of breast cancer and facial cancer are life-threatening and once rejected, leave patients with soft tissue defects and disfiguration. Mastectomy and tumor resection surgeries are examples of non-elective surgical procedures that mandate the replacement of lost soft tissue to restore physical shape and/or physiological function. Facial reconstructive surgeries are necessary after cancer resections such as basal cell carcinoma, squamous cell carcinoma, melanoma and other head and neck cancers. Improved medical managements of cancer have prolonged survival rate, with the result that cancer survivors wish to have the morphology and function of their soft tissue defects restored. Injuries take place during war, leading to soft tissue trauma in addition to skeletal injuries. Work-related accidents and traffic accidents are additional causes for soft tissue defects. Soft tissue is missing after bums, necessitating the reconstruction of not only the skin, but also subcutaneous soft tissue. In congenital anomalies such as hemifacial microsomia (one half of the face under-developed relative to the other half), a considerable amount of soft tissue is missing and needs to be reconstructed. In 2003, a total of approximately 6 million individuals in the U.S. received reconstructive surgical procedures performed by plastic surgeons (American Society of Plastic Surgeons http://www.plasticsurgery.org/public_education/2004Statistics.cfm).

In the United States, about $5 billion was spent on surgical cosmetic procedures and about $2 billion on non-surgical cosmetic procedures (such as collagen injections) in 2002. This correlates to about 7 million cosmetic surgical and non-surgical procedures performed that year alone.

For decades, surgeons have utilized their creative wisdom to improve soft tissue reconstruction procedures. Autologous soft tissue grafts and synthetic materials are predominant practice in soft tissue reconstruction procedures. Each approach has its own pros and cons, and is suitable for a specific type of patient and soft tissue defects based on clinical judgment. Unfortunately, the current procedures for soft tissue reconstruction and augmentation fall short of the ideal, with adverse side effects such as donor site morbidity, leakage, extrusion, unnatural texturing, resorption, and immune rejection. Although autologous mature adipose tissue has been used as a filler for soft tissue defects, the results are unpredictable. Volume reduction appears to be a primary concern with autologous grafts resorbing up to 70%. Volume reduction of autologous soft tissue grafts may be due to apoptosis of the transplanted mature adipocytes due to their low tolerance to ischemia and slow revascularization rate. Lipectomy or liposuction procedures provide another route of fat transplantation. Single cell suspensions of mature adipocytes isolated from liposuction aspirate have been incorporated in scaffolds and transplanted to fill soft tissue defects. However, volume reduction remains an issue, likely because 85% of the cytoplasm of mature adipocytes is lipid, leading to a tremendous need for angiogenesis. In most severe cases, fat grafts from liposuction aspirates are associated with suboptimal blood supply and necrosis of the graft. Mechanical stresses resulting from liposuction are projected to damage up to 90% of the adipocytes upon implantation. Another complicating factor is that mature adipocytes are fully differentiated and do not proliferate, leading to a shortage of adipogenic cells in fat grafts.

Pre-adipocytes that can be isolated from animal or human fat tissue via excision or liposuction procedures are preferable to mature adipocytes because pre-adipocytes are believed to be capable of proliferating to a certain extent *ex vivo,* and tolerating hypoxic environments to a greater extent than mature adipocytes. However, some pre-adipocytes are likely damaged during excision and/or liposuction procedures. Several pre-adipocyte cell lines such as 3T3-L1 and 3T3-F442A have been used toward engineering adipose tissues in biomaterials such as PGA meshes. These adipogenic cell lines, despite their considerable value for *in vitro* studies, are immortalized and thus not as valuable as primary pre-adipocytes for *in vivo* engineering of adipose tissue. For example, pre-adipocytes seeded in porous PLA scaffolds generate adipose tissue *in vivo.* Peptide-linked alginate implants supported the adhesion and proliferation of seeded sheep pre-adipocytes, and adipose tissue formation. Exogenously delivered bFGF to human and murine pre-adipocytes was reported to promote continuous adipogenic differentiation and adipose tissue formation in collagen or Matrigel scaffolds.

Mesenchymal stem cells derive from embryonic stem cells and differentiate into cell lineages that form all connective tissues such as adipose tissue, cartilage, bone, skeletal muscles and interstitial fibrous tissue. MSCs can be isolated from bone marrow, adipose tissue, deciduous teeth, and skeletal muscle. In addition to their multipotent nature, MSCs can self-renew and replicate many passages without losing their stemness. MSCs were first isolated and identified as fibroblast-like cells that are adherent to cell culture Petri dishes. In humans, MSCs are typically aspirated from the bone marrow of the superior iliac crest, whereas murine MSCs are commonly aspirated from the mid-diaphysis of the tibia and femur. Upon exposure to various established induction media such as adipogenic, chondrogenic, osteogenic, myogenic, etc, MSCs differentiate into distinct pathways and begin to express genes and protein markers characteristics of corresponding cell lineages.

Mesenchymal stem cells (MSCs) are progenitors of adipogenic cells and adipocytes. End-stage adipocytes neither replicate nor further differentiate. Thus, a shortage of adipose tissue-forming cells contributes, among other factors, to volume reduction after current soft tissue grafting procedures. Human mesenchymal stem cells (hMSCs) were reported to differentiate into adipogenic cells in monolayer culture of Petri dishes. A large number of genes expressed during adipogenesis of human MSCs *in vitro* have been delineated.

A scaffold material is usually required to provide initial adhesion and support for tissue-forming cells in engineered grafts. Selecting the appropriate scaffold for a particular tissue engineering application is important for the success of the endeavor. Tissue development is dependent on the structural environment, cell-biomaterial interaction, and potentially biological signals incorporated in the scaffold.

Previous work on cell-based adipose tissue engineering has primarily utilized porous scaffolds. The advantage of porous scaffolds is their potential to facilitate vascular ingrowth. However, a common drawback of porous scaffolds in adipose tissue engineering is premature resorption and/or degradation of the porous scaffold, potentially associated with enzymes produced by invading host cells. Hydrogels encompass a broad arena of scaffolds including collagen, elastin, hyaluronic acid, agarose, alginate, chitosan, and polyethylene glycol Hydrogels. PEG hydrogels have been used to encapsulate various cell types, including chondrocytes and osteoblasts. The rheological and recovery properties of PEGDA hydrogel are reported to share several parameters comparable to human abdominal adipose tissue.

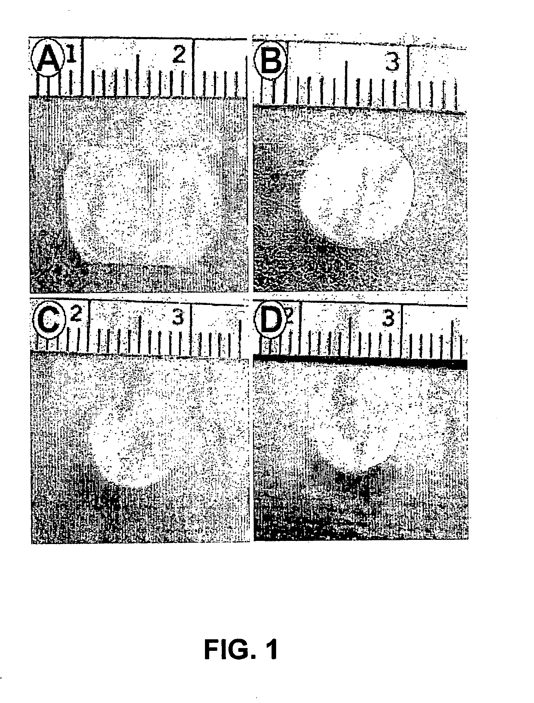

Reconstructive and plastic surgeries focuses on specific shapes and dimensions. Hypothetically, a successfully tissue-engineered kidney from stem cells, when realized, does not need to have the precise shape as the patient's normal kidney so long as the engineered kidney functions well *in vivo.* In contrast, soft tissue defects as illustrated in FIGS. 1A and B must be restored to have the original shape and dimensions, in addition to having physiological function. Volume reduction can be as severe as up to 60% over time. In fact, "post-operative volume reduction" of soft tissue grafts as a key issue among other complications of soft tissue reconstruction procedures in surgical literature.

Although volume reduction is likely caused by a multitude of factors such as scarring or suboptimal tissue integration, the key issue is hypothesized to be a shortage of incorporated cells that are capable of 1) sustained synthesis of adipose and fibrous matrices to maintain the volume, and 2) self replication to replenish the supply of adipose and fibrous matrix-forming cells. End-stage cells such as adipocytes cannot meet these needs.

A major obstacle in the application of biological materials in tissue engineering is suboptimal tissue ingrowth and poor vascularization. Biomaterials that have been used *in vivo* owe their success to the avascular nature of the replacement host tissues such as cartilage and skin. Both native cartilage and skin are largely avascular and sufficiently thin for the diffusion of nutrients in the absence of vascularization. However, survival of tissue forming cells *in vivo* is generally poor for engineered tissues or organs with dimensions greater than a few millimeters, yet, larger masses of tissue-forming cells are required to engineer most tissues and organs. Accordingly, neovascularization is necessary to promote the survival of tissue-forming cells engineered tissues and organs.

Blood vessels are proposed to form by two different biological processes in nature, vasculogenesis and angiogenesis. Vasculogenesis is a process of capillary formation from endothelial progenitor cells. Angiogenesis, the formation of new blood vessels from pre-existing blood vessels, is a vital and necessary process in both tissue development and wound healing. Angiogenesis takes place potentially by two mechanisms: intussusception and sprouting. Intussusceptive angiogenesis occurs when interstitial cellular columns migrate into a pre-existing blood vessel. This type of angiogenesis causes remodeling of the local vascular network. Sprouting angiogenesis, on the other hand, refers to the formation of new blood vessels from existing vascular structures. Although vasculogenesis has recently been observed postnatally, angiogenesis is the common process leading to the formation of new blood vessels in the adult. Both angiogenesis and vasculogenesis involve a complex cascade of events that require numerous signaling processes and involve multiple cell lineages. Although the process of vascularization of natural tissues is increasingly illustrated, our understanding of neovascularization of engineered tissues is still at its infancy.

Tissue engineering has employed primarily two approaches to induce vascularization: stimulating endogenous angiogenic responses and engineering physical structures in biomaterials that mimic the natural vascular network. Quiescent endothelial cells can be stimulated to convert into angiogenic endothelial cells by a number of angiogenic molecules such as vascular endothelial growth factor (VEGF), hepatocyte growth factor (HGF), and fibroblast growth factor (FGF). In addition to its angiogenic actions, bFGF has been shown to stimulate the proliferation of osteoblastic cells, making bFGF especially important for bone tissue engineering.

Microchannels have been patterned in tissue-engineering materials by photolithography to simulate capillaries. Despite previous intense efforts on promoting angiogenesis in a number of biocompatible polymer materials, *in vivo* neovascularization in nonporous hydrogel scaffolds remains a challenge for tissue engineering applications.

Poly(ethylene glycol) (PEG) is among a number of hydrogel polymers that have been used in tissue engineering. PEG is a non-toxic and water soluble polymer which is not recognized by the immune system. PEG has already been approved by the Federal Drug Administration (FDA) for biomedical applications in humans. An advantage of PEG is its moldability into a given shape and dimensions that are necessary for engineering tissues and organs. PEG hydrogels can be fabricated using a photoinitiator, which results in crosslinking of nodes of oligoacrylate connected by linear PEG. However, PEG is a dense hydrogel and has slow degradation rates, which can be regarded as an advantage and disadvantage, pending upon specific tissue engineering applications. For drug delivery, PEG's slow degradation rate is helpful in allowing controlled release of encapsulated cytokines. Cytokine diffusion rate can be modified by varying the molecular weights and concentration of PEG hydrogels. As a scaffold for tissue engineering, PEG degradation rate can be modified with regard to its crosslinking, concentration, and copolymerization. For example, the degradation rate of PEG is altered by copolymerizing with poly-d-1-lactic-co-glycolic acid (PLGA). However, a pending issue with widespread use of PEG for *in vivo* tissue engineering applications is slow and minimal amount of host tissue ingrowth and vascularization in PEG hydrogel.

Hong et al, Annals of Biomedical Engineering, 2005; Vol. 33: 511-517, discloses the seeding of MSCs on biodegradable gelatine sponges with subsequent adipogenic differentiation to form regenerated adipose tissue.

Alhadlaq et al, Tissue Engineering, 2005; Vol. 11: 556-566, discloses engineered adipose tissue from human MSCs using a hydrogel system. The invention was to encapsulate the stem cells in the polymer.

### SUMMARY

Biologically engineered soft tissue derived from adult mesenchymal stem cells within a biocompatible scaffold has a predetermined 3-dimensional configuration; i.e., shape and size or dimensions. Biologically engineered soft tissue constructs are prepared in any shape or size from adult mesenchymal stem cells and are readily made *in vitro, ex vivo,* and in *vivo.* These implants are useful to patients who require cosmetic surgery due to aesthetics, disease, congenital anomaly, or trauma.

Mesenchymal stem cells in hydrogels with various supplements provide implants with reduced volume loss, low rate of cell degradation, and moldability to predetermined shape and dimensions.

Methods and compositions are provided for preparing biologically engineered soft tissue having a predefined (or predetermined) shape and dimensions *in vivo* or *ex vivo* by adult mesenchymal stem cells within a biocompatible scaffold. In accordance with this method, mesenchymal stem cells (MSCs) are provided dispersed in a biocompatible scaffold of predetermined 3-dimensional configuration. Those cells are induced to differentiate into adipose and fibroblastic tissue-forming cells by contacting the MSCs with an adipogenic inducing supplement and a fibroblast growth factor bFGF. That contact is maintained for a time period sufficient for at least some adult mesenchymal stem cells to differentiate into soft tissue having the predetermined 3-dimensional configuration as determined by the nature of the reconstruction or repair needed. The constructs are soft tissue implanted into a recipient in need thereof by standard surgical procedures. Adipocytes and fibroblasts are present in the formed soft tissue, with stem cells being present in small to undetectable quantities.

A biocompatible scaffold is constructed from a hydrogel polymer such as polymerized polyethylene glycol diacrylate. The biocompatible scaffold, and therefore the engineered soft tissue prepared using that scaffold, have a predetermined 3-dimensional configuration. The hydrogel polymer can be formed before, or after, dispersal of MSCs within a biocompatible scaffold precursor such that the polymer entraps the MSCs. The hydrogel polymer is formed by, for example, photopolymerization of the biocompatible scaffold precursor.

PEG hydrogel is advantageous as a scaffold for adipose tissue engineering applications. PEG is hydrophilic, biocompatible and undergoes slow degradation which is useful for volume retention of the engineered adipose tissue.

Immunorejection of the construct is prevented because tissue-forming cells are from the patient's own bone marrow or other sources which are compatible. Soft tissue integrates with the patient's own tissue biologically driven healing process. Current orthopedic surgical approaches can be utilized alleviating the need for separate institutional approval. Moreover, adult stem cells are used (instead of embryonic stem cells) so that ethical tissues are minimized.

Standard cell culture procedures are modified so that a large number of tissue engineering laboratories can be instructed to make biologically-engineered soft tissues.

Materials and methods described herein represent a rare combination of physical and chemical approaches to coerce vascular tissue ingrowth *in vivo* into a nonporous biocompatible hydrogel. Vessel-like structures lined by endothelial-like cells in the PEG hydrogel contain red blood cells and are stained positively to both anti-VEGF and WGA antibodies, indicating the presence of endothelial cells in the infiltrated host tissue. In combination with the morphology of red blood cells contained in the lumens lined by endothelial-like cells within engineered macrochannels, tissue ingrowth in the present study is likely vascular tissue. Suggesting that physical macrochannels larger than microchannels used previously, and a chemical stimulant, basic fibroblast growth factor, either alone or in combination, promotes vascular tissue ingrowth in PEG hydrogel *in vivo.* However, important differences exist between the effects of macrochannels and bFGF. In comparison with random infiltration of host vascular tissue by bFGF-loaded PEG without macrochannels, vascular and granular tissue ingrowth is directed to within the lumen of the macrochannels of bFGF-loaded PEG without infiltrating the rest of the PEG. Thus, the capacity of apparently random host tissue invasion induced by bFGF in PEG hydrogel, which may be undesirable for tissue engineering applications, is coerced into guided vascular tissue ingrowth by engineered macrochannels. This directed vascular tissue ingrowth is significant in generic tissue engineering applications where angiogenesis is needed. Although macrochanneled PEG without bFGF induces vascular tissue ingrowth within the channels, the amount of vascular tissue volume infiltrated into macrochanneled, bFGF-loaded PEG is significantly more than macrochanneled PEG without bFGF.

According to a first aspect of the invention there is a soft tissue construct comprising adult mesenchymal stem cells dispersed in a scaffold constructed from a nonporous biocompatible hydrogel polymer, wherein the scaffold further comprises basic fibroblast growth factor (bFGF) and an adipogenic agent capable of stimulating differentiation of the stem cells to an adipocyte cell, the scaffold having a three dimensional configuration that comprises macrochannels of a size sufficient to promote vascular tissue in growth.
According to a second aspect of the invention there is a method of for preparing a soft tissue construct having a predetermined three dimensional shape and dimensions, the method comprising:
(a) providing adult mesenchymal stem cells dispersed in a scaffold constructed from a non-porous biocompatible hydrogel polymer, the scaffold having predetermined three dimensional configurations, comprising macrochannels of a size sufficient to promote vascular tissue in-growth; and
(b) contacting the adult mesenchymal stem cells with an adipogenic inducing supplement and bFGF sufficient to differentiate the adult mesenchymal stem cells to adipocyte cells and Fibroblastic-tissue forming cells;
wherein the soft tissue construct is suitable for implanting into a host.

### BRIEF DESCRIPTION OF THE DRAWINGS

FIG. 1 is a series of four photographs (A-D) that illustrate the gross appearance of tissue-engineered fat-tissue constructs and control constructs after 4-week subcutaneous implantation in the dorsum of immunodeficient mice: (A) Plastic mold used to load the polymer/cell suspension during fabrication of constructs; (B) Tissue-engineering fat-tissue construct encapsulating adipogenic-treated hMSCs retained shape and dimensions of the original mold; (C) Harvested control construct encapsulating untreated hMSCs; (D) Harvested control construct with no cells encapsulated. Note dimensional resemblance between all constructs and the original mold contrast between tissue-engineered construct (B) and control constructs (C and D).

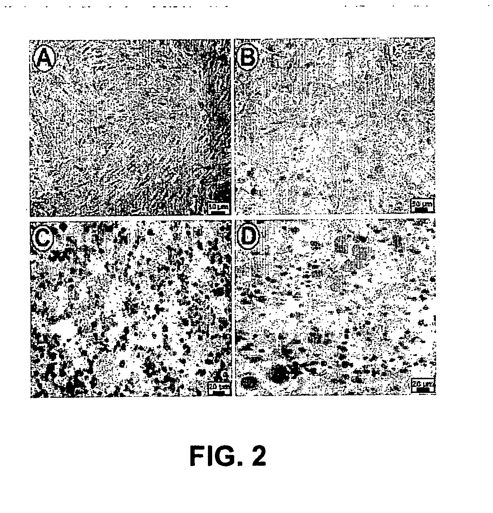

FIG. 2 is a series of four photomicrographs (A-D) illustrating adipogenesis by human mesenchymal stem cells (hMSCs) maintained *ex vivo* in monolayer cultures and hydrogel constructs; (A) Positive staining with Oil Red-O of a monolayer culture of hMSCs maintained with adipogenic medium for 4 weeks; (B) Matching control monolayer culture for (A) demonstrating negative reaction to Oil Red-O staining when hMSCs were maintained with basic medium for 4 weeks; (C) Fat deposits demonstrated by positive staining with Oil Red-O in this representative micrograph for hydrogel constructs encapsulating adipogenic-treated hMSCs and maintained *ex vivo* for 4 weeks in adipogenic medium; (D) Representative micrograph of a matching control for (C) showing no reaction to Oil Red-O.

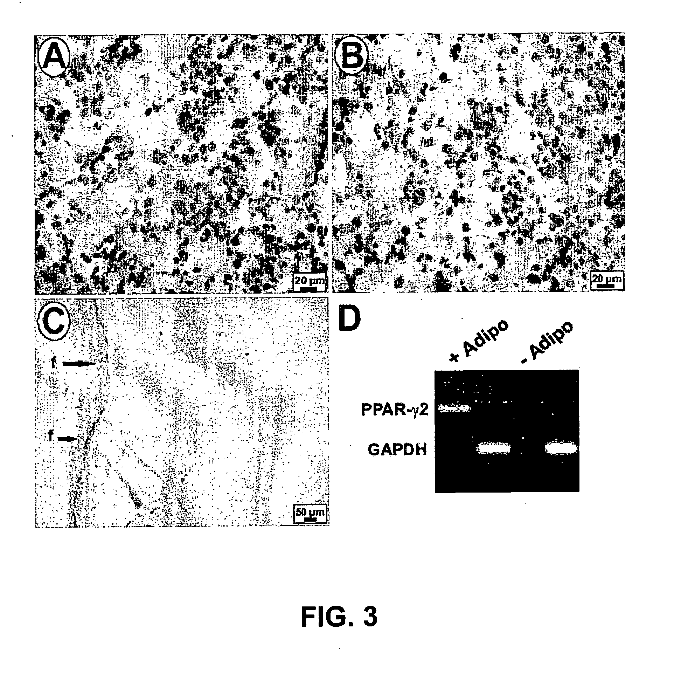

FIG. 3 is a series of four photomicrographs (A-D) that show adipogenesis by hMSCs maintained *in vivo* in tissue-engineered constructs by histochemical staining and RT-PCR; (A) Positive staining with Oil Red-O in tissue-engineered hydrogel construct encapsulating adipogenic-treated hMSCs and implanted in the dorsum of immunodeficient mouse for 4 weeks; (B) Representative micrograph of control hydrogel constructs encapsulating untreated hMSCs and implanted in the dorsum of immunodeficient mice for 4 weeks demonstrated no fat deposition as evident by the negative reaction to Oil Red-O staining; (C) Representative micrograph of control hydrogel constructs encapsulating no cells showing negative reaction to Oil Red-O staining and intact border with fibrous capsule surrounding the construct and absence of host-cell invasion for the construct; (D) RT-PCR analysis showing expression of adipocyte-specific gene (PPAR-γ2) in constructs, harvested after 4 weeks of subcutaneous implantation in immunodeficient mice, encapsulating adipogenic-treated hMSCs (+Adipo) or untreated hMSCs (-Adipo) relative to a housekeeping gene (GAPDH).

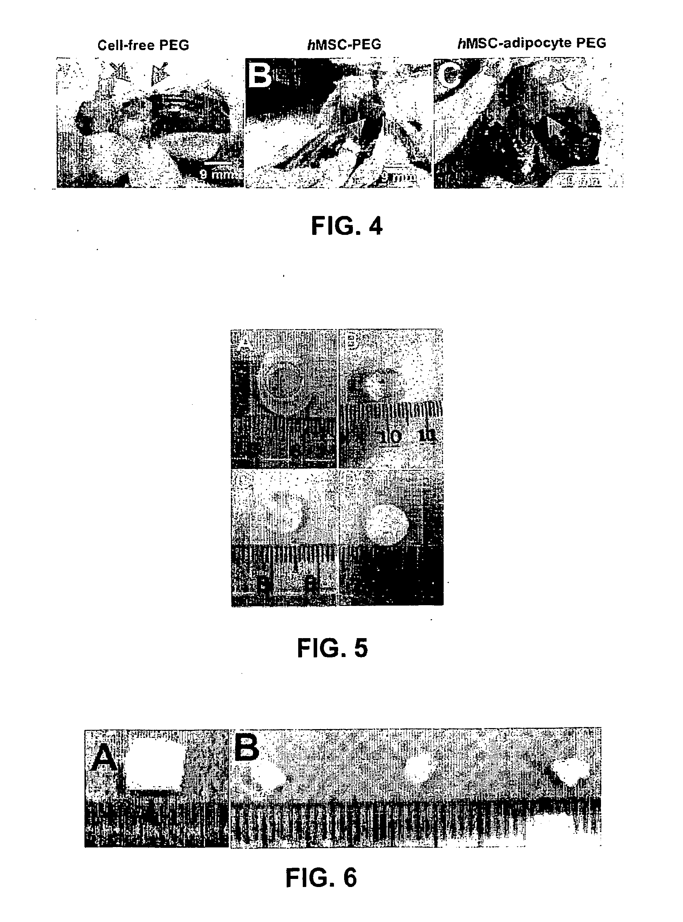

FIG. 4 Harvest of hMSC-derived adipose tissue in PEG hydrogel and control groups after *in vivo* implantation. A: Cell-free PEG hydrogel (between arrows). B: PEG hydrogel encapsulating hMSCs (no adipogenic differentiation) (between arrows). C: Engineered adipose tissue in PEG hydrogel encapsulating hMSC-derived adipogenic cells (between arrows) showing its adhesion to surrounding host tissue and retention of the original 9 mm diameter (greater magnification than A and B).

FIG. 5 Shape, dimensions and photo-opaqueness of engineered adipose tissue and controls: (A) Plastic cap of Eppendorf tubes (9 mm dia.) that was used as the generic mold of the shape and dimensions; (B) Harvested cell-free PEG hydrogel is largely transparent; (C) PEG hydrogel encapsulating hMSCs (no adipogenic differentiation) showing some photo-opacity; (D) PEG hydrogel encapsulating hMSC-derived adipogenic cells showing substantial photo-opacity and maintained the pre-defined shape and dimensions.

FIG. 6 Shape and dimensions of *in vivo* harvested collagen sponges seeded with hMSCs and control groups showing loss of both original shape and dimensions. (A) Sterile collagen sponges trimmed into approx. 4×4×4 mm cubes; (B) Harvested cell-free collagen sponge, seeded with hMSCs, and hMSC-derived adipocytes (from left sequentially) after 4-wk in vivo implantation showing substantial reductions in size and substantial shape changes.

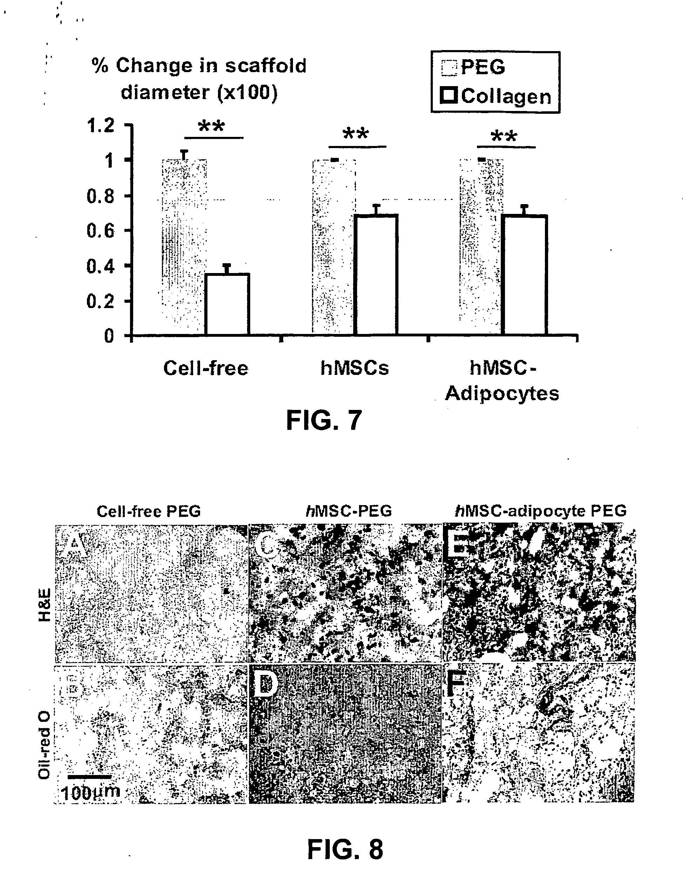

FIG. 7 Quantitative changes in scaffold diameters (×100% ± S.D.) of harvested PEG and collagen grafts after 4 wks of in vivo implantation. hMSCs: human mesenchymal stem cells. Gray histograms represent PEG grafts, whereas empty histograms represent collagen grafts. Cell-free PEG constructs (N=6), PEG hydrogel constructs encapsulating hMSCs (N=6), or encapsulating hMSC-derived adipocytes (N=6) maintained the original diameter of the scaffold nearly 100%. In contrast, collagen sponges without seeded cells (N=4) lost the original diameter by 65 0%±5.0, whereas collagen sponges seeded with hMSCs (N=4) and with hMSC-derived adipocytes (N=6) lost the original diameters by 31.7%±5.8 and 31.7%±5.4, respectively. **: P<0.01.

FIG. 8 H&E and Oil-red O staining of engineered adipose tissue and controls after 4-wk *in vivo* implantation (A); hMSCs: human mesenchymal stem cells; (B) Cell-free PEG hydrogel showing neither resident cells nor lipid vacuoles; (C) PEG hydrogel encapsulating hMSCs without adipogenic differentiation showing abundant cells; (D) Oil-red O staining of hMSC PEG hydrogel showing no lipid vacuoles; (E) PEG hydrogel encapsulating hMSC-derived adipogenic cells showing abundant resident cells among islands of space; (F) Oil-red O staining of PEG hydrogel encapsulating hMSC-derived adipogenic cells showing abundant lipid vacuoles among flattened cells that resemble adipocytes. 10× magnification.

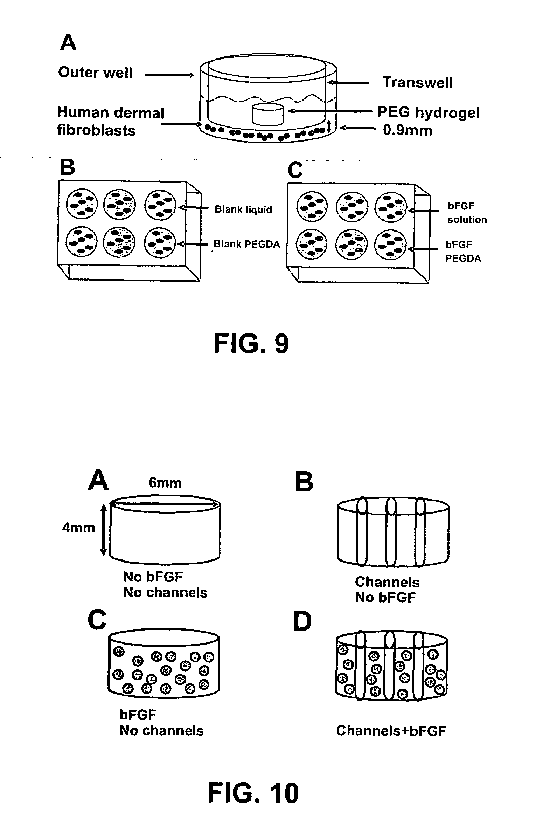

FIG. 9 shows a schematic representation of fibroblast growth factor-basic (bFGF)-loaded PEG hydrogel cylinders and human dermal fibroblast (hDFs) coatings.

FIG. 10 is a schematic representation of an *in vivo* implantation of bFGF-loaded PEG hydrogel and creation of macrochannels.

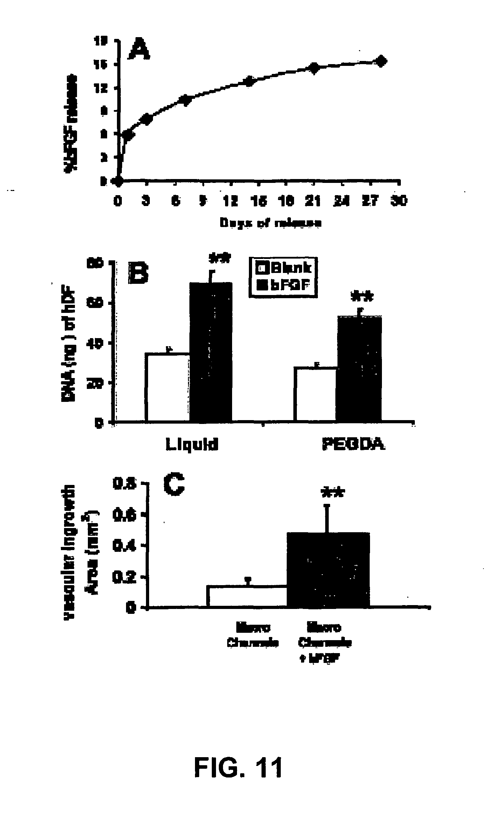

FIG. 11 shows an *in vivo* release profile of bFGF encapsulated in PEG hydrogel.

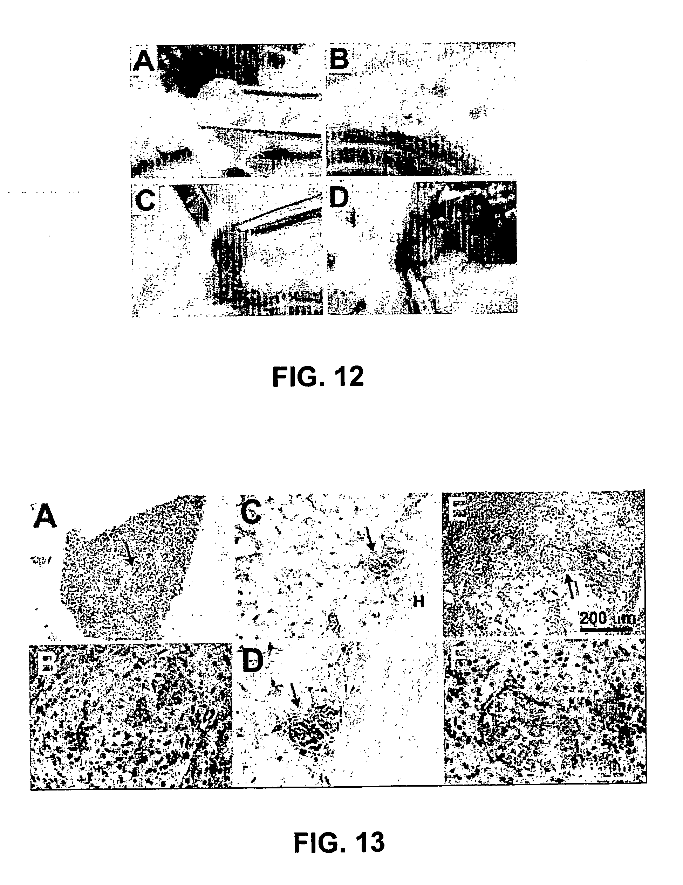

FIG. 12 shows a harvest of *in vivo* implanted samples: (A) PEG hydrogels without cells, bFGF, or channels showing a lack of macroscopic host tissue invasion; (B) PEG hydrogels with 3 macrochannels (1 mm diameter each) showing host tissue in growth in the 3 lumen of created macrochannels; (C) PEG hydrogels loaded with bFGF but without macrochannels; (D) PEG hydrogel with both bFGF and macrochannels showing general red color and 3 host tissue in-growth in the 3 lumen of created macrochannels.

FIG. 13 shows high magnifications of *in vivo* harvested PEG hydrogels treated with different physical and chemical conditions, i.e., bFGF with or without macrochannels, demonstrating the formation of blood vessel-like structures.

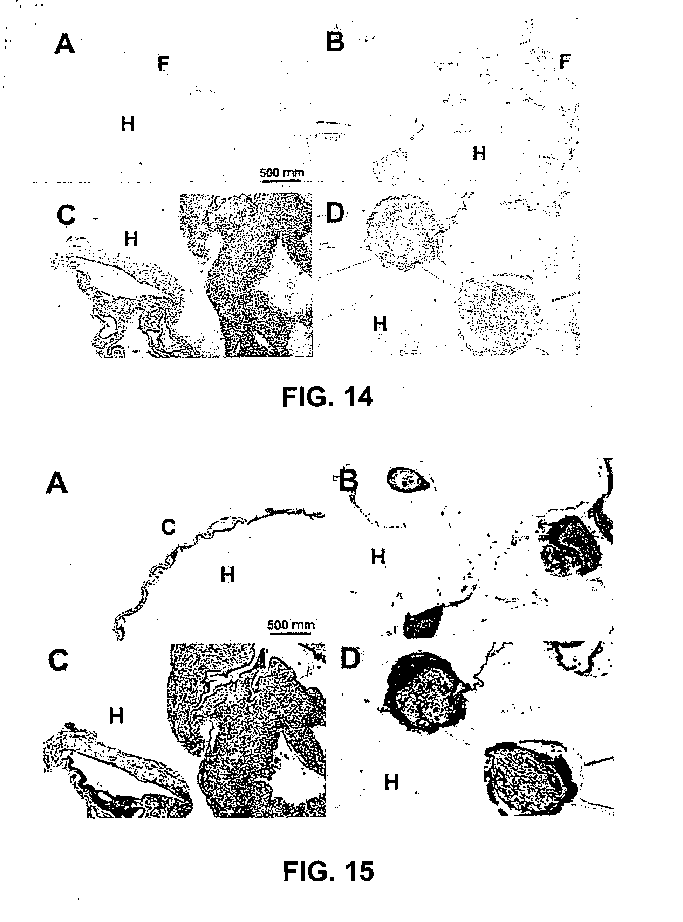

FIG. 14 shows an anti-VEGF antibody based negative immunoblotting on a representative section of a harvested PEG hydrogel cylinder without either bFGF or macrochannels.

FIG. 15 shows the positive staining of the bulk of the invading host tissue with lectin from *Triticum vulgaris* (wheat germ agglutinin) (WGA).

### DETAILED DESCRIPTION OF THE DISCLOSURE

Soft tissue is a key structure to restore in reconstructive and augmentative surgeries. Current materials for soft tissue reconstruction and/or augmentation suffer from shortcomings such as suboptimal volume retention, donor site morbidity, and poor biocompatibility. Biologically viable soft tissue can be engineered by taking a tea-spoon full of the patient's adult stem cells, expanding them, differentiating them into adipogenic cells and encapsulating them into appropriate biocompatible polymer materials. The end result is a minimal donor site trauma such as needle size, immune compatibility because patient's own stem cells are used, and long-term volume maintenance because stem cells are capable of replenishing adipogenic cells for retaining the pre-defined shape and dimensions of the engineered soft tissue. Stem cell derived soft tissue grafts are realizable in plastic and reconstructive surgical procedures.

*De novo* synthesis of soft tissue prepared from adult mesenchymal stem cells (MSCs) within a biocompatible scaffold of predetermined 3-dimensional configuration provides constructs for repair, augmentation or reconstruction of soft tissue. Adult MSCs are capable of differentiating into all connective tissue-forming cell lineages including cartilaginous, osseous, and adipose tissues. MSCs can be obtained with minimally invasive procedures from bone marrow or other connective tissue sources in the body, are highly expandable in culture, and can be readily induced to differentiate into adipose tissue-forming cells after exposure to a well-established adipogenic inducing supplement (Pittenger *et al.,* Caplan, 2003). Moreover, the use of the adult MSCs is advantageous over the use of embryonic stem cells or differentiated adipocytes for both procedural and ethical reasons.

Adult mesenchymal stem cells are derived from bone marrow cells. At least some adult mesenchymal stem cells are differentiated into adipocytes so that a mixture of both cell types is initially present that changes over time to substantially only adipocytes, with stem cells being present in small to undetectable quantities. Adipose tissue is fabricated *in vivo* by the stem cells or prepared *ex vivo* by the stem cells.

MSCs are placed within scaffolding material for adipose tissue engineering. The placement of the MSCs within the scaffold can occur after the scaffold is prepared e.g. as where stem cells diffuse from the host body into the prepared scaffold. MSCs are admixed with a precursor of the scaffold, and the scaffold is then constructed around the MSCs, thereby capturing at least some of those cells within the scaffold network. The scaffold is biocompatible, supports tissue formation, and exhibits sufficient stability to permit successful integration between the newly formed tissue and the surrounding host tissue.

Illustrative host animals include laboratory animals such as rat, mouse and rabbit, a companion animal such as a dog or cat, a veterinary animal such as a horse (equine), cow (bovine), sheep (ovine) or goat (caprine), or a primate such as a monkey, ape (chimp, orangntan or gorilla) or man.

Hydrogel polymers provide tissue-forming cells with an environment mimicking the extracellular matrix in the body. Hydrogel polymers are hydrophilic, three-dimensional networks that absorb or adsorb (sorb) large amounts of water or biological fluids, while maintaining their distinct three-dimensional structure. Hydrogel polymers such as alginate, coral, agarose, fibrin, collages, bone, cartilate, hydroxyapatite, calcium phosphate, polylactic acid (PLA), polyglycolic acid (PGA) or their copolymer (PLGA), chitosan, and polyethylene glycol-based polymers (peg-based polymers) such as polyethylene glycol diacrylate, polyethylene glycol dimethacrylate and mixtures thereof are suitable. A scaffold is formed by polymerization of polyethylene glycol diacrylate monomer [MW 3400; Shearwater Polymers, Huntsville, AL]. A polyethylene glycol diacrylate or dimethacrylate monomer can have a molecular weight of about 1000 to about 100,000 daltons and about 2000 to about 5000 daltons.

Polyethylene glycol-based hydrogel polymers have certain advantages for tissue engineering applications because of their proven biocompatiblity and their demonstrated capacity to support growth and differentiation of MSCs into multiple lineages such as bone. Also, the possibility of administering the cell-polymer system under the skin with a minimally invasive procedure (injection) and the ability of these hydrogels to undergo transdermal radiation-induced polymerization such as photopolymerization. Doses of X- or gamma-radiation that are non-injurious to the host animal can also be used to initiate polymerization after implantation into a host animal.

Biologically engineered adipose tissue derived from adult mesenchymal stem cells within a biocompatible scaffold preferably has a predetermined 3-dimensional configuration; i.e., shape and size. That configuration can be obtained by polymerizing (forming) the biocompatible scaffold in a mold or other form that is ex vivo (outside of the host's body). The 3-dimensional configuration of the biocompatible scaffold can also be determined by the size and shape of the *in vivo* locus within the host's body at which the biocompatible scaffold is prepared.

An adipogenic construct from which adipose tissue is fabricated includes biocompatible scaffold and adult mesenchymal stem cells derived from bone marrow. This construct includes an adipogenic agent dispersed within the scaffold. The adipogenic agent may be proglitazone, growth factors of the β family, prostaglandins, ciglitazone, dexamethasone or a mixture thereof.

A composition includes a biocompatible scaffold wherein the scaffold is comprised of hydrated, hydrogel polymer such as polymerized polyethylene glycol diacrylate, adult mesenchymal stem cells derived from human bone marrow, and further includes an adipogenic agent, a nutrient medium, basic fibroblast growth factor, optionally at least one antibiotic. Exemplary adipogenic agents, nutrients and antibiotics include amphotericin B, ciglitazone, biotin, dexamethasone, gentamicin, insulin, 3-isobutyl-1-methylxanthine, and L-thyroxine.

Disclosed is a method of producing biologically engineered soft tissue *in vivo* includes implanting a composition comprising a biocompatible scaffold that contains adult mesenchymal stem cells and an adipogenic medium into a host animal. The adult mesenchymal stem cells are attached to the scaffold as by entrapment following polymerization of a mixture that includes the cells and a monomer precurser such as polyethylene glycol diacrylate and also contains amphotericin B, ciglitazone, biotin, dexamethasone, gentamicin, insulin, 3-isobutyl-1-methylxanthine and L-thyroxine. The biocompatible scaffold formed on polymerization includes a polymerized, hydrated hydrogel polymer.

Additional steps include harvesting adult mesenchymal stem cells, contacting the cells with adipogenic medium and maintaining the contact for a time sufficient to induce differentiation into adipocytes, loading the adipocytes onto a biocompatible scaffold, and implanting the scaffold loaded with the adipocytes into a host animal.

A method of producing biologically engineered soft tissue *ex vivo* includes attaching adult Mesenchymal stem cells to a biocompatible scaffold wherein the scaffold is effected by entrapment of the cells within the scaffold, hydrogel matrix that also contains an adipogenic agent, bFGF a nutrient medium, and optionally at least one antibiotic. The scaffold may include a polymerized, hydrated polyethylene glycol diacrylate matrix that contains the adipogenic agent, bFGF, nutrient medium, and optionally at least one antibiotic that comprise amphotericin B, ciglitazone, biotin, dexamethasone, gentamicin, insulin, 3-isobutyl-1-methylxanthine, and L-thyroxine.

Commercially available human mesenchymal stem cells, from an anonymous adult donor, were differentiated into adipogenic cells hMSC-derived adipocytes were encapsulated in PEG hydrogel and implanted into surgically created subcutaneous pockets in the dorsum of immunodeficient mice. After 4-wk in vivo implantation, stem cell derived adipose tissue grafts were harvested. FIG. 4 demonstrates the retrieval process of PEG hydrogel scaffolds. The PEG hydrogel encapsulating hMSC-derived adipogenic cells, such as those in FIG. 1D, appeared darker in color and well attached to the host subcutaneous tissue (FIG. 5). FIG. 6 demonstrates the shape, dimensions, and different photo-opaqueness of PEG adipose tissue grafts after *in vivo* implantation. The PEG-hydrogel construct encapsulating hMSC-derived adipogenic cells not only maintained the original shape and dimensions, but also demonstrated considerable photo-opaqueness (FIG. 6D), suggesting that a substantial amount of biological structures had been formed in the hMSC-derived adipogenic PEG construct. In contrast to the PEG hydrogel constructs, collagen sponges harvested after the same 4-wk *in vivo* implantation lost their original shape and dimensions. As seen in FIG. 7, all collagen sponges, whether cell-free, seeded with hMSCs or hMSC-derived adipogenic cells, lost the original shape and shrunk a substantial amount (FIG. 7C). Oil-Red-O staining, a histological marker for lipid accumulation, of hMSC-derived adipogenic PEG and collagen constructs demonstrated positive staining. However, H&E staining showed no host cell invasion into the PEG constructs, suggesting that all the generated adipose tissue was from the implanted human MSC-derived adipogenic cells. In comparison, there are likely host mouse cells invading collagen sponges, leaving the possibility of host cell contribution to adipogenesis within the collagen constructs (FIG. 8).

Human mesenchymal stem cells readily differentiate into components of soft tissue not only in monolayer culture of Petri dishes, but also in 3D hydrogel and in *vivo* in animal models. Human MSCs are readily isolated from the patients whose soft tissue defects need surgical repair, so that autologous cell therapy is available to circumvent issues of immunorejection. hMSC-derived soft tissue grafts in PEG hydrogel are capable of maintaining their pre-defined shape and dimensions virtually 100% following 4-wk subcutaneous implantation.

Angiogenesis remains a challenge for engineered tissues including adipose tissue engineering. Despite isolated attempts, controlled angiogenesis needs to be induced in engineered soft tissue grafts. Angiogenesis in fat pads derived from pre-adipocyte cell lines is from the host, instead of pre-adipocytes, indicating the need to stimulate host angiogenesis. Although adipose tissue is the primary component of soft tissue, soft tissue consists of more than adipose tissue. For example, host fibrous tissue may be needed in engineered soft tissue grafts. Several approaches for inducing angiogenesis in bone tissue engineering are useful for enabling angiogenesis in engineered adipose tissue.

Scale up is a common tissue engineering challenge including the engineering of soft tissue. The disclosed engineered soft tissue graft is up to 9 mm in diameter and is sufficient for certain facial soft tissue defects and other small soft tissue defects. However, soft tissue defects after mastectomy and large trauma demand multiple fold scale ups. Issues such as mass transport, nutrient diffusion, angiogenesis and nerve innervation are increasingly challenging upon scaling up of engineered soft tissue.

### EXAMPLES

**Example 1: *Ex vivo* incubation and in vivo implants for adipose tissue engineering.**

### A. Isolation and culture of human MSCs

Human MSCs (hMSCs) were isolated from whole bone marrow aspirates from the iliac crest of a healthy 22-year-old male donor (AllCells, LLC., Berkely, CA). Briefly, cells were enriched by adding RosetteSep mesenchymal enrichment cocktail (StemCell Technologies, Inc., Vancouver, Canada) to the whole bone marrow sample. The cells were then diluted with twice the volume of phosphate-buffered saline (PBS) containing 2 percent fetal bovine serum (FBS) (Atlanta Biologicals, Inc., Norcross, GA) and 1 mM ethylenediamine tetraacetic acid (EDTA) (Sigma, St. Louis, MO).

The diluted sample was layered on top of a Ficoll-Paque (StemCell Technologies, Inc., Vancouver, Canada) cushion and centrifuged for 25 minutes at 300 x g. Enriched cells were removed from the ficoll-Paque plasma interface, washed with PBS containing 2 percent FBS and 1 mM EDTA, and cells were resuspended in complete culture medium: MesenCult basal medium for hMSCs (StemCell Technologies, Inc., Vancouver, Canada), 10 percent human mesenchymal stem cell stimulatory supplements (StemCell Technologies, Inc., Vancouver, Canada), and 1 percent antibiotic-antimycotic (Gibco, Carlsbad, CA). Cells were counted, plated in 10 ml complete medium in 100-mm culture dishes, and maintained in 95 percent air/5 percent CO₂ at 37°C. After 24 hours, non-adherent cells were discarded; adherent cells were washed twice with PBS and maintained for 10-14 days with fresh medium change every 3-4 days. When large colonies were formed (typically 10-14 days) and before confluence, primary hMSCs were trypsinized with 0.25 percent trypsin and 1mM EDTA for 5 minutes at 37°C, counted, and replated in 100-mm dishes. Only first-passage hMSCs were used.

### B. Treatment of hMSCs with adipogenic, supplements

For encapsulation studies, first-passage hMSCs were cultured separately in basic or adipogenic medium for 1 week. Basic medium consisted of MesenCult basal medium for human mesenchymal stem cells (StemCell Technologies, Inc., Vancouver, Canada), 10 percent human mesenchymal stem cell stimulatory supplements (StemCell Technologies, Inc., Vancouver, Canada), and 1 percent antibiotic-antimycotic (Gibco, Carlsbad, CA). Adipogenic medium consisted of MesenCult basal medium for human mesenchymal stem cells (StemCell Technologies, Inc., Vancouver, Canada), 10 percent human adipogenic stimulatory supplements (StemCell Technologies, Inc., Vancouver, Canada), and 1 percent antibiotic-antimycotic (Gibco, Carlsbad, CA). Cultures were maintained in 95 percent air/5 percent CO₂ at 37°C with medium changes every 3-4 days.

To demonstrate the adipogenic process in monolayer culture, first-passage hMSCs were cultured separately in basic or adipogenic medium for 4 weeks and stained with Oil Red-O (Sigma, St. Louis, MO). Cultures were compared for positive Oil-Red O staining indicating adipose deposits in the culture.

### C. Hydrogel/photoinitiator solution preparation and construct fabrication

To prepare the hydrogel solution, poly(ethylene glycol) diacrylate (PEGDA) (MW 3400; Shearwater Polymers, Huntsville, AL) was dissolved in sterile PBS supplemented with 100 U/ml penicillin and 100 µg/ml strepomycin (Gibco, Carlsbad, CA) to a final solution of 10 percent w/v. A photoinitiator, 2-hydroxy-1[4-(hydroxyethoxy) phenyl1]-2-methyl-1-propanone (Ciba, Tarrytown, NY), was added to the PEGDA solution to obtain a final photoinitiator concentration of 0.05 percent w/v.

After one-week of maintenance in either basic or adipogenic medium, first-passage hMSCs were trypsinized with 0.25 percent trypsin and 1mM EDTA for 5 minutes at 73° C, counted, and resuspended separately in PEGDA polymer/photoinitiator solution at a density of 5/106 cell/ml. To prepare each construct, a 150 µl aliquot of cell/polymer/photoinitiator suspension was loaded into 9 mm diameter plastic inserts (caps of 1.5 ml microcentrifuge tubes) (Fisher Scientific, Hampton, NH). After loading, plastic inserts carrying cell/polymer/photoinitiator suspensions were exposed to long-wave, 365 nm ultraviolet lamp (Glowmark, Upper Saddle River, NJ at an intensity of about 4 mW/cm2 for 5 minutes. The photopolymerized constructs were then removed from the plastic inserts, washed twice with sterile PBS, and transferred to 6-well culture plates (Fisher Scientific, Hampton, NH) with the corresponding, basic or adipogenic, medium.

### D. Ex vivo incubation and in vivo implantation of constructs

For the *ex vivo* study, hydrogel constructs encapsulating hMSC-derived adipogenic cells (N-12) were maintained with adipogenic medium (described herein), while hydrogel constructs encapsulating untreated hMSCs (N=12)m were maintained with basic medium (described herein). All cultures were maintained in 95 percent air/5 percent CO₂ at 37 °C for 4 weeks, changing to fresh medium every 3-4 days.

For *in vivo* studies, the polymerized hydrogel constructs encapsulating adipogenic-treated hMSCs (N=8), constructs enscapulating adipogenic-treated hMSCs (N=4), and control constructs with no cells (N=4) were removed from the molds and washed twice with sterile PBS. All constructs were implanted in subcutaneous pockets in the dorsum of severe combined immunodeficient (SCID) mice (4 constructs/mouse) (Harlan, Indianapolis, IN) prepared by blunt dissection under general anethesia with IP injection of 100 mg/kg ketamine plus 5 mg/kg xylazine/

### E. Cell Viability Assay

Cell viability was assessed in 4-week *ex vivo* maintained constructs using live/dead viability/cytotoxicity kit (Molecular Probes, L-3224, Eugene, OR) following the manufacturer's recommended protocol. Calcein (a fluorescent dye) diffuses through cell membrane and reacts with intracellular esterase to the produce green fluorescence, whereas ethidium homodimer diffuses only through damaged cell membrane and binds to nucleic acids to produce red fluorescence. After cell labeling, samples were viewed under an inverted optical microscope (Leica Microsystems, Wetzler, Germany) equipped with longpass dual-emission fluorescent filter (Chroma, Rockingham, VT).

### F. Histology

Following 4-week *ex vivo* maintenance and *in vivo* cultivation in the dorsum of SDID mice, constructs were embedded in optimum cutting temperature (OCT) tissue freezing mixture (IMEB, Chicago, IL) and 10 µm sections were cut using standard histological technique. Section were stained with Oil Red-O (Sigma, St. Louis, MO), mounted with glycerol gelatin (Sigma, St. Louis, MO), and viewed under a light microscope (Leica Microsystems, Weltzer, Germany).

### G. RNA extraction and reverse, transcription-polymerase chain reaction

Total RNA was extracted from the harvested *in vivo* constructs using the RNeasy Purification Reagent (Qiagen; Valencia, CA). The constructs were homogenized (pelled pestle mixer; Kimble/Kontes, Vineland, NJ) in 1.5 ml microcentrifuge tubes containing 200 µ of RLT buffer from the RNeasy kit. Then, 400 µ1 RLT buffer was added, followed by further homogenization with the QUIshredder (Qiagen, Valencia, CA) column. Reverse transcription (RT) was performed using random hexamers with the superscript amplification system (Gibco, Carlsbad, CA). One-microliter aliquots of the resulting cDNA were amplified in 50 µl volume at annealing temperature of 58°C for 30 cycles using the Ex Taq DNA Polymerase Premix (Takara Bio, Otsu, Shiga, Japan). Polymerase chain reaction (PCR) was performed using the specific human primers of PPAR-γ2, sense: 5'-GCTGTTATGGGT GAAACTCTG-3' (SEQ ID NO:1), antisense: 5'- ATAAGGT-GGAGATGCAGGCTC-3' (SEQ ID NO.: 2), and the housekeeping gene glyceraldehyde-3-phosphate dehydrogenase (GAPDH) , SENSE: 5' - CCCATGTTCGTCA-TGGGTGT-3' (SEQ ID NO: 3)" antisense: 5'-TGGTCATG-AGTCCTTCCACGATA-3' (SEQ ID NO.: 4). PCR was performed for 30-35 cycles, with each cycle consisting of denaturing at 95°C for 30 seconds, annealing at 55-60°C for 30 seconds and elongating at 72°C for 1 minute, with an additional 10-minute maintenance at 72°C after completion of the last cycle. To exclude possible contamination of genomic DNA, PCR was also applied to reactions without RT. The amplified complementary DNA was electrophoresed through a 1 percent agarose gel, stained, and photographed under ultraviolet light.

### H. Gross examination of tissue-engineered adipose tissue constructs

Following 4 weeks of *in vivo* implantation and *ex vivo* maintenance, both constructs (encapsulating hMSC-derived adipogenic cells) and control constructs (encapsulating untreated hMSCs or no cells) maintained their physical shape and dimensions of the original plastic mold (FIG. 1A). Upon physical manipulation, the constructs were firm, elastic and unyielding to a limited degree. The tissue-engineered adipogenic constructs (FIG. 1B) appeared light yellow in color and photo-opaque in contrast to transparent constructs harvested from *in vivo* implantation containing hMSCs treated with basic medium (but not adipogenic medium) (FIG. 1C) and harvested control construct with no encapsulated cells (FIG 1D)

### 1. Adipogenesis by hMSCs in ex vivo maintained monolayer cultures and hydrogel constructs

Human MSC monolayer cultures maintained with adipogenic medium for 4 weeks demonstrated positive reaction to Oil Red-O staining indicated by the red color (FIG. 2A), in comparison with lack of Oil Red-O staining of hMSCs cultured in monolayer in the presence of basic medium (and without adipogenic medium). Oil Red-O is a lysochrome (fat soluble dye) with deeper red color, predominantly used for demonstrating triglycerides in frozen sections (Lillie, Conn's Biological Stains, 9th Ed. Baltimore Williams & Wilkins co., 1977). Similarly, hydrogel constructs encapsulating adipogenic-treated hMSCs showed positive staining with Oil Red-O after 4 weeks of *ex vivo* maintenance in PEG-based hydrogel in comparison with a lack of Oil Red-O staining in PEG-based hydrogel encapsulating non-adipogenic treated hMSCs (FIG. 2C AND D).

### J. Tissue-engineered adipogenic constructs in vivo

Histological and RT-PCR analyses demonstrated *de novo* formation of adipose tissue in the hydrogel constructs encapsulating adipogenic-treated hMSCs after 4 weeks of *in vivo* cultivation in the dorsum of SCID mice (FIG. 3). Adipogenic constructs showed positive Oil Red-O staining (FIG. 3A) relative to the control constructs encapsulating untreated hMSCs (FIG. 3B) or those with no cells encapsulated (FIG. 3C). In addition, control constructs with no cells encapsulated showed no signs of host cells invasion and intact borders of the hydrogel construct (FIG. 3C). The adipocyte-specific PPAR-γ2 gene was positively expressed in the adipose-tissue constructs encapsulating adipogenic-treated hMSCs (FIG. 3D). The mice were housed in a temperature and light controlled room (23-25°C, 14 hours light/day, and given standard daily amounts of food and water.

### Example 2: Engineered soft tissue in growth and angiogenesis in non-porous biomatierals.

### A. PEG Hydrogel Preparation

Poly(ethylene glycol) diacrylate (1 g) (Shearwater Huntsville, AL, USA) was dissolved in 6.6 mL PBS supplemented with 133 units/mL penicillin and 133 mg/mL streptomycin (Invitrogen, Carlsbad, CA, USA) to a final solution of 6.6% w/v.

An ultraviolet photoinitiator, 2-hydroxy-1-[4-(hydroxyethoxy) phenyl]-2-methyl-1-propanone (Irgacure) (Ciba, Tarrytown, NY, USA), was dissolved in 100% ethanol at a concentration of 50 mg/mL. A final unit stock PEG solution was prepared by adding 100 µL photoinitiator/ethanol solution to 6.6 mL PEG solution.

### B. bFGF Release Profile In Vitro

Fibroblast growth factor-basic (bFGF) human recombinant (Sigma, St. Louis, MI, USA) was reconstituted per manufacturer guidelines with PBS containing 0.1 % bovine serum albumin (BSA) at a concentration of 25 µg/mL. This solution was further diluted with PBS supplemented with BSA to a final concentration of 0.4 µg/mL. A total of 100 µL 0.4 µg/mL bFGF solution was added to a sterile test tube with 200 µL PEG stock solution. This mixture was pipetted 5 times to ensure homogeneity and then into 75 µL aliquots into cylindrical molds of the Eppendorf cap. These cylindrical molds were photopolymerized with UV light at 365 nm for 5 min (Glo-Mark, Upper Saddle River, NJ, USA). The bFGF-loaded PEG hydrogel cylinders in separate test tubes, each containing 1 mL of 0.1 % BSA in PBS, were placed in 37°C water bath for 1,3, 7, 14, 21, and 28 days (N=4 per time point) to measure bFGF release kinetics. At each time point, the supernatant of each test tube was assayed using a human bFGF immunoassay (R&D Systems, Minneapolis, MN, USA) (Alhadlaq et al., 2004; Moioli et al., 2005).

### C. Bioactivity of Released bFGF from PEG Hydrogel

Fibroblast growth factor-basic (bFGF) human recombinant (Sigma, St. Louis, MI, USA) was reconstituted as described herein, and further diluted to a final concentration of 4 µg/mL. A total of 200 µL of 4 µg/mL bFGF solution was added to a sterile test tube along with 400 µL of PEG stock solution. This mixture was pipetted 5 times to ensure homogeneity and then into 75 µL aliquots into Eppendorf cylindrical molds, followed by photopolymerization with UV light at 365 nm for 5 min (Glo-Mark, Upper Saddle River, NJ, USA). To prepare bFGF-free PEG hydrogel cylinders, 200 mL of 0.1 % BSA in PBS was used in place of bFGF solution.

Human dermal fibroblast (hDFs) (Cambrex, Walkersville, MD, USA) were plated at a density of 20,000 cells/well in 4 mL of Dulbecco's modified eagle medium supplemented with 10% fetal bovine serum and 1% penicillin and streptomysin (DMEM complete). The adherent hDFs were incubated in the following 4 conditions (N=6 per group): 1) blank liquid containing 0.1% BSA in PBS (FIG. 9A), 2) blank PEG hydrogel loaded with 0.1% BSA in PBS (FIG. 9A), 3) bFGF solution containing 6.25 ng/mL bFGF without PEG hydrogel (FIG. 9B), and 4) bFGF-loaded PEG hydrogel containing 0.1 µg bFGF (FIG. 9B). For Group 4, the hDFs were incubated in DMEM with transwell inserts (Becton Dickinson Labware, Franklin Lakes, NJ) placed in each cell culture well (0.4 µm pore size) at 37°C and with 5% CO₂ for 1 wk (FIG. 9C). The transwell inserts were placed 0.9 mm above the monolayer culture of hDFs in DMEM medium, thus exposing the cells to released bFGF without direct contact with bFGF-loaded PEG cylinders (FIG. 9C). After 1-wk incubation under each of the above 4 conditions, the hDFs were harvested by treatment with 500 µL 0.1 % Triton X solution (Sigma, St. Louis, MI, USA), scraped using a sterile disposable cell lifter (Fisherbrand, Pittsburgh, PA), collected in tissue culture tubes, lysed and homogenized on ice using sonication (Sonica Dismembrator Mode 100, Fisher, Pittsburgh, PA, USA). The hDF lysate was then collected in a centrifuge tube and stored at -80°C. DNA content was measured using fluorescent quantification of Hoechst 33258 bound to double-stranded DNA per our prior method (Fluorescent DNA Quantitation kit, Bio-Rad, Hercules, CA) (Alhadlaq et al., 2004; Moioli et al., 2005).

### D. In Vivo Implantation of bFGF-loaded PEG Hydrogel

A total of 100 µL 1 µg/µL bFGF solution was added to 200 µL PEG solution, followed by gentle mixing, and then pipetted in 75 µL aliquots into cylindrical mold of an Eppendorf cap. The resulting PEG cylinders were photopolymerized with UV light at 365 nm for 5 min (Glo-Mark, Upper Saddle River, NJ, USA). A total of 3 macrochannels were created in each PEG hydrogel cylinder by inducing 3 punctures with a sterile capillary tube (FIGS. 10B and D). The following PEG hydrogel cylinders were prepared for *in vivo* implantation (N=4 per group): 1) blanks (bFGF-free and macrochannel-free) (FIG. 10A), 2) bFGF loaded but without macrochannels (FIG. 10B), 3) with macrochannels but without bFGF (FIG. 10C), and 4) bFGF loaded and with macrochannels (FIG. 10D).

Male severe combined immune deficiency (SCID) mice (strain C.B17; 4-5 wk old) were anesthetized with intraperitoneal injection of ketamine (1 00mg/kg body weight) and xylazine (4mg/kg body weight) (Alhadlaq et al., 2004). The mouse dorsum was clipped of hair and placed in a prone position, followed by disinfection with 10% povidone iodine and 70% alcohol. A 1 cm-long linear cut was made along the upper midsagittal line of the dorsum, followed by blunt dissection to create subcutaneous pouches. Each SCID mouse received 4 PEG hydrogel implants: blank PEG gel, bFGF-loaded PEG without macrochannels, macrochanneled PEG without bFGF, and PEG with both bFGF and macrochannels. The incision was closed with absorbable plain gut 4-0 sutures. All PEG hydrogel cylinders were implanted in vivo for 4 wks. The present animal protocol was approved by the institutional animal care committee.

### E. Harvest of In Vivo PEG hydrogel Samples, Histology, Inimunohistochemistry and Data Analysis

Four weeks following subcutaneous implantation in the dorsum of SCID mice, PEG hydrogel cylinders were harvested. Following CO₂ asphyxiation, an incision was made aseptically in the dorsum of the SCID mouse. Following careful removal of the surrounding fibrous capsule, PEG hydrogel cylinders were isolated from the host, rinsed with PBS, and fixed in 10% formalin for a minimum of 24 hrs. The PEG samples were then embedded in paraffin and sectioned in the transverse plane (transverse to macrochannels; c.f., FIG. 10B and 10D) at 5 µm thickness. Paraffin sections were stained with hematoxylin and eosin.

Sequential sections adjacent to H&E stained sections were deparaffinized, washed in PBS, and digested for 30 min at room temperature with bovine testicular hyaluronidase (1600 U/ml) in sodium acetate buffer pH 5.5 with 150 mM sodium chloride. All immunohistochemistry procedures followed previous methods (Mao et al., 1998; Alhadlaq and Mao, 2005). Briefly, sections were treated with 5% bovine serum albumin (BSA) for 20 min at room temperature to block nonspecific reactions. The following antibodies were used: anti-vascular endothelial growth factor (anti-VEGF) (ABcam, Cambridge, MA USA), and biotin-labeled lectin from Triticum vulgaris (wheat germ agglutinin) (WGA) with and without its inhibitor, actyleuraminic acid (Sigma, St. Louis, MI, USA). WGA binds to carbohydrate groups of vascular endothelial cells rich in α-D-GlcNAc and NeuAc (Jinga et al., 2000; Izumi et al., 2003). After overnight incubation with primary antibodies in a humidity chamber, sections were rinsed with PBS and incubated with IgG anti-mouse secondary antibody (1:500; Antibodies, Davis, CA) for 30 min. Sections were then incubated with streptavidin-HRP conjugate for 30 min in humidity chamber. After washing in PBS, the double linking procedure with the secondary antibody was repeated. Slides were developed with diaminobenzadine (DAB) solution and counterstained with Mayer's hematoxylin for 3 to 5 min. Counterstained slides were dehydrated in graded ethanol and cleared in xylene. The same procedures were performed for negative controls except for the omission of the primary antibody.

The amount of tissue ingrowth in the engineered macrochannels was quantified by manually outlining the amount of infiltrated host tissue in macrochanneled PEG hydrogels with or without loaded bFGF on H&E stained sections under 4x magnification. Automated area values generated by computerized image analysis were compared using the Student t tests and ANOVA to determine whether the amount of tissue ingrowth differed significantly between macrochanneled PEG hydrogels with and without loaded bFGF.

### F. In Vitro bFGF Release Profile

The release profile of bFGF encapsulated in PEG hydrogel was demonstrated in FIG. 11A. The present release kinetics of bFGF encapsulated in PEG showed a burst release (approximately 5.8%) within the first 24 hrs, followed by sustained release up to the tested 28 days (FIG. 11A). By 28 days, a total of 15.3% of the encapsulated bFGF was released from PEG hydrogels (FIG. 11A).

### G. bFGF Bioactivity upon Release from PEG

The encapsulated bFGF release from PEG showed distinctive effects on human dermal fibroblasts in monolayer culture. The DNA content of hDFs treated with bFGF solution at 69.8±5.6 ng (meant±S.E.; N=6; solid left histogram in FIG. 11B) was significantly higher than the DNA content of hDFs without exposure to bFGF at 34.5±2.7 ng (N=6; 'Blank' or the left empty histogram in FIG. 11B). Similarly, the DNA content of hDFs treated with bFGF released from PEG hydrogel at 52.6±3.7 (N=6; solid right histogram in FIG. 11B) was significantly higher than the DNA content of hDFs exposed to bFGF-free PEG hydrogels at 27.5±1.7 (N=6; 'Blank' or the right empty histogram in FIG. 11B). Interestingly, the DNA content of hDFs cultured in DMEM supplemented with bFGF solution was significantly higher than the DNA content of hDFs cultured in DMEM supplemented with bFGF released from PEG hydrogels (P=0.43) (two solid histograms in FIG. 11B). The differences in DNA contents of hDFs induced by bFGF solution and bFGF released from PEG hydrogel will be discussed herein.

### H. PEG Hydrogel Harvested from In Vivo with Macrochannels and/or bFGF

Upon 4-wk *in vivo* implantation in the dorsum of SCID mice, representative H&E stained section of a harvested PEG hydrogel cylinder with neither macrochannels nor bFGF showed no host tissue ingrowth (FIG. 12A). However, macrochanneled PEG hydrogel without bFGF demonstrated host tissue ingrowth only into the lumen of macrochannels, and not in the rest of PEG (FIG. 12B). In contrast, bFGF-loaded PEG hydrogel without macrochannels demonstrated apparently random areas of host tissue ingrowth in isolated areas of PEG hydrogel (FIG. 12C). Most interestingly, bFGF-loaded and macrochanneled PEG hydrogel demonstrated host tissue ingrowth into the lumen of macrochannels, but not the rest of PEG (FIG. 12D). The PEG hydrogel cylinders with both macrochannels and loaded with bFGF showed significantly more ingrowing host tissue at 0.47±0.18 mm² than the bFGF-free PEG hydrogel cylinders with macrochannels at 0.13±0.05 mm² (mcan±S.D.; P<0.01; N=4 per group), indicating more host tissue ingrowth into macrochanneled PEG hydrogel with bFGF than without bFGF.

High magnifications of *in vivo* harvested PEG hydrogels treated with different physical and chemical conditions, i.e., bFGF with or without macrochannels, demonstrated the formation of blood vessel-like structures (FIG. 13). The *in vivo* harvested PEG hydrogel with macrochannels but without bFGF showed widely distributed blood vessel-like structures under 20 × magnification (FIG. 13A). Under 60 × magnification, small blood vessel-like structures were apparently lined by endothelial cell-like cells and contained eosin-positive cells that closely resembled red blood cells (FIG. 13B). In contrast, bFGF-loaded PEG hydrogel without macrochannels showed widespread cellular infiltration in apparently random fashion, with regions of PEG hydrogel still present (H in FIG. 13C). The multiple foam-like structures in FIG. 13C likely represented PEG hydrogel undergoing degradation along invaded host cells and tissues (c.f. a large piece of PEG hydrogel labeled with H in FIG. 13C). Under 60 × magnification, a band of thick membrane-like structure with high cellularity separated PEG hydrogel from the invaded host tissues (FIG. 13D). Characteristically, PEG hydrogel with both macrochannels and loaded with bFGF demonstrated intensive host cell invasion among apparent blood vessel-like structures with an example labeled with arrow in FIG. 13E. Under 60 × magnification, the blood vessel-like structure labeled with arrow showed well-defined lining by endothelial-like cells, and contained donut-shaped, nucleus-free cells that closely resembled red blood cells (FIG. 13F).

Anti-VEGF antibody showed negative immunoblotting on the representative section of a harvested PEG hydrogel cylinder without either bFGF or macrochannels (FIG. 14A). However, PEG hydrogel with macrochannels but without bFGF demonstrated somewhat faint reaction only in macrochannels, and not in the rest of PEG (FIG. 14B). In contrast, PEG hydrogel loaded with bFGF but without macrochannels demonstrated widespread anti-VEGF immunoblotting, although there remained areas of apparent hydrogel scaffold with the absence of tissue ingrowth (FIG. 14C). PEG hydrogel with both macrochannels and loaded with bFGF demonstrated positive anti-VEGF staining in macrochannels, but not the rest of PEG (FIG. 14D).

The bulk of the invading host tissue was stained positive with lectin from *Triticum vulgaris* (wheat germ agglutinin) (WGA) (FIG. 15), an antibody that labels vascular endothelial cells (Jinga et al., 2000; Izumi et al., 2003). Staining with WGA inhibitor, actyleuraminic acid, confirmed this positive staining with WGA. The positively WGA stained band in FIG. 15A represented a membrane surrounding, formed by host tissue, of the implanted macrochannel-free and bFGF-free PEG hydrogel cylinder. Intense WGA staining was present in not only PEG hydrogel cylinder without bFGF but with macrochannels (FIG. 15B), but also PEG hydrogel cylinder with both bFGF and macrochannels (FIG. 15D), suggesting the presence of endothelial cells in the invaded host vascular and granular tissues. In contrast, WGA staining of the representative PEG hydrogel cylinder loaded with bFGF but without macrochannels was somewhat less intense (FIG. 15C).

### DOCUMENTS CITED

Alcantar, NA., Aydil, ES., Israelachvili, JN (2000) JBiomed Mater Res 51(3):343-351.
Alhadlaq A, Mao JJ (2003) J Dent Res 82:951-956.
Alhadlaq A, Mao JJ (2004) Stem Cells and Development 13:436-448.
Alhadlaq A, Tang M, Mao JJ (2005) Tissue Engineering 11:556-566.
Alster et al., Plast. Reconstr. Surg. (2000), 105: 2515-2525
Atala A (1999) Urol Clin North Am 26:157-168.
Atala, (1999); Walgenbach et al., (2001), Anat. Rec. 263: 372-378;
Billings E, May, J (1989) Plast Reconstr Surg. 83:368-381.
Brey and Patrick, Jr., (2000) IEEE Eng. Med. Biol. Mag. 19:122;
Brey EM, Patrick, CW Jr (2000) IEEE Eng Med Biol Mag 5:122-125.
Bryant, SJ, Anseth, KS (2003) J Biomed Mater Res 64:70-79.
Burdick and Anseth, (2002) Biomaterials 23:4315;
Butler et al., (1998) Plast. Reconstr. Surg.102:161;
Caplan, (2003) Novartis Found. Symp. 249:17
Caplan, AI (1991) J Orthop Res 9: 641.
Coleman SR (1995) Aesth Plast Surg 19:421-429.
Coleman, (1995) Aesth. Plast. Surg. 19:421;
Coppit GL, Lin DT, Burkey BB (2004) Curr Opin Otolaryngol Head Neck Surg 12:281-287.
Davis RE, Guida RA, Cook TA (1995) Arch Otolaryngol Head Neck Surg 121:95-100.
Duranti et al., (1998) Dermatol. Surg 24:1317;
Elisseeff et al., (1999) Proc. Natl. Acad. Sci. U.SA. 96:3104;
Eppley, (1999) Plast. Reconstr. Surg. 104:1761;
Fischbach C, et al. (2004a) Exp CellRes 300:54-64.
Friedenstein, AJ, Petrakova, KV (1966) J Embry Exp Morp 16;381-390.
Fu et al., 2002 Biomaterials 23:4425;
Garfein ES, Orgill DP, Pribaz JJ (2003) Clin Plast Surg 4:485-98.
Garfein et al., (2003) Clin. Plast. Surg. 30:485).
Gath et al., (2002) Plast. Reconstr. Surg. 109:889.
Griffith LG, Naughton G (2002) Science 295:1009-1014.
Halberstadt et al., (2002) Tissue Eng. 8(3):309-319.
Halberstadt, C, et al. (2002) Tissue Eng 8:309-19.
Hart, (2003) Plast. Surg. Nurs. 23:55).
Hoffman, (2002) Adv. Drug Deliv. Rev. 54:3
Kaban LB, Padwa BL, Mulliken JB (1998) J Oral Maxillofac Surg 56:628-638.
Katz et al., 1999 Clin Plast. Surg. 26:587;
Kim et al, (2003) Osteoarthritis Cartilage 11:653;
Kimura Y, Ozeki M, Inamoto T, Tabata Y (2003) Biomaterials 14:2513-21.
Kononas, TC, Bucky, LP, Hurley, C, Amy, JWJ (1993) Plast Reconstr Surg 91:763-768.
Kral et al, (1999) Plas. Reconstr.Surg 104(6):1732-1738;
Langer RS, Vacanti JP (1999) Sci Am 280:86-89.
Lee and Mooney, (2001) Chem. Rev. 101:1869
Lee et al., in Lanza, et al. (Eds.), Principles of Tissue Engineering, 2nd Ed. San Diego: Academic Press, 2000: 409-423;
Lee KY, Halberstadt CR, Holder WD, Mooney DJ (2000) In R. P. Lanza, R. Langer, and J. Vacanti (Eds.), Principles of Tissue Engineering, 2nd Ed. San Diego: Academic Press, 2000. Pp. 409-423.
Lorenz et al., 2000 Plast. Reconstr. Surg. 105:2467;
Mao JJ (2005) Biol Cell 97:289-301.
Marler et al., 2000, Plast. Reconstr. Surg. 105:2049-2058;
Marler et al., 2000; Patrick, Jr., 2000, Surg. Oncol. 19:302-311;
Marler JJ, et al. (2000) Plast Reconstr Surg 105:2049-2057.
Martens, P, Bryant, SJ., Anseth,KS (2003) Biomacromolecules 2:283-292.
Monahan R, et al. (2001) JAm Dent Assoc 132:1402-1408.
Neels JG, Thinnes T, Loskutoff DJ (2004) FASEB J 18:983-985.
Nguyen and West, (2002) Biomaterials 23:4307
Oxley et al., (1993) Biomaterials 14:1064;
Patel PN, Smith CK, Patrick CW Jr (2005) J Biomed Mater Res A 73A:313-319.
Patrick CW Jr (2000). Semin Surg Oncol 3:302-11.
Patrick CW Jr, Chauvin PB, Reece GP (1999) Tissue Eng 5:139.
Patrick CW Jr, Friedrich J, Miller MJ (1998) Anal Ouant Cytol Histol 3:199-206.
Patrick Jr., (2000) Surg. Oncol. 19:302-311
Patrick Jr., Frontiers in Tissue Engineering, 1st Ed., Oxford, Elsevier Science, 1998: 369-382;
Patrick, CW Jr., Miller, MJ (2003) Clin Plast Surg 1: 91-103.
Patrick, Jr. et al, (1999) Tissue Eng. 5:139;
Patrick, Jr., (2000) Surg. Oncol. 19:302-311;
Patrick, Jr., (2001) Anat. Rec. 263:361-366;
Patrick, Jr., Frontiers in Tissue Engineering, 1st Ed., Oxford, Elsevier Science, 1998: 369-382;
Peterson SL, Moore EE (2003) Plast Reconstr Surg 112:1371-1375.
Pittenger et al., (1999) Science 284:143-147;
Pittenger MF, Mackay AM, Beck SC, Jaiswal RK, Douglas R, Mosca JD, Moorman MA,
Poshusta and Anseth, (2001) Cells Tissues Organs 169:272;
Rahaman MN, Mao JJ (2004) Biotechnol Bioeng (In press).
Roncevic R, Roncevic D (1999) J Ci-aiziofac Surg 10:284-300.
Shin H, Temenoff JS, Bowden GC, Zygourakis K, Farach-Carson MC, Yaszemski MJ, Mikos AG (2005) Biomaterials 26:3645-3654.
Simonetti DW, Craig S,Marshak DR (1999) Science 284:143-147.
Smaehel J, Meyer V, Shutz K (1990) Eur J Plast Surg 13:163-168.
Thaller SR, Zarem HA, Kawamoto HK (1987) Am J Surg 154:149-153.
Toriyama K, Kawaguchi N, Kitoh J, Tajima R, Inou K, Kitagawa Y, Torii S (2002) Tissue Eng 1:157-65.
Tremain N, Korkko J, Ibberson D (2001) Stem Cells 19:408-418.
von Heimburg D, Zachariah S, Heschel I, Kuhling H, Schoof H, Hafemann B, Pallua N (2001). Biomaterials 22:429-438.
Vural E (2004) Curr Oncol Rep 6:133-140.
Walgenbach et al., (2003) Clin. Plast. Surg. 30:485;
Walgenbach et al., (2001), Anat. Rec. 263: 372-378
Williams et al., (2003) Tissue Eng. 9:679
Yaremchuk MJ (2003) Plast Reconstr Surg111 :1818-1827. Alhadlaq and Mao 2003 .J. Dent. Res. 82:951

## Claims

1. A soft tissue construct comprising adult mesenchymal stem cells dispersed in a scaffold constructed from a nonporous biocompatible hydrogel polymer, wherein the scaffold further comprises basic fibroblast growth factor (bFGF) and an adipogenic agent capable of stimulating differentiation of the stem cells to an adipocyte cell, the scaffold having a three dimensional configuration that comprises macrochannels of a size sufficient to promote vascular tissue in-growth.

2. The soft tissue construct of claim 1, further characterized as having a predefined three dimensional shape and dimension.

3. The soft tissue construct of claim 1, wherein the hydrogel polymer is polyethylene glycol diacrylate, polyethylene glycol dimethacrylate, or mixtures thereof.

4. The soft tissue construct of claim 3, wherein the hydrogel polymer consists of polyethylene glycol diacrylate.

5. The soft tissue construct of claim 1, comprising adipose and fibrous tissue.

6. The soft tissue construct of claim 1, wherein the adult mesenchymal stem cells are derived from bone marrow cells.

7. The soft tissue construct of claim 1, wherein the scaffold comprises a material selected from the group consisting of polylactic acid, polyglycolic acid, a copolymer of polylactic acid-polyglycolic acid, alginate, chitosan, agarose, fibrin and collagen.

8. The soft tissue construct of claim 1, wherein the adipogenic agent is selected from the group consisting of proglitazone, dexamethasone, insulin, 3-isobutyl-1 -methylxanthine, and L-thyroxine.

9. A method for preparing a soft tissue construct having a predetermined three dimensional shape and dimensions, the method comprising:
(a) providing adult mesenchymal stem cells dispersed in a scaffold constructed from a non-porous biocompatible hydrogel polymer, the scaffold having predetermined three dimensional configurations, comprising macrochannels of a size sufficient to promote vascular tissue in-growth; and
(b) contacting the adult mesenchymal stem cells with an adipogenic inducing supplement and bFGF, sufficient to differentiate the adult mesenchymal stem cells to adipocyte cells and fibroblastic-tissue forming cells;
wherein the soft tissue construct is suitable for implanting into a host.

10. The method of 9, wherein the hydrogel polymer is polyethylene glycol diacrylate.

11. The method of any one of claims 9 to 10, wherein the scaffold comprises a material selected from the group consisting of polylactic acid, polyglycolic acid, a copolymer of polylactic acid-polyglycolic acid, alginate, chitosan, agarose, fibrin and collagen.

12. The soft tissue construct of any one of claims 1 to 8 for use in the repair, augmentation, or reconstruction of soft tissue of a host.

13. The use of claim 12, where the host is a mammal.

## Patentansprüche

1. Weichteilkonstrukt, das adulte mesenchymale Stammzellen umfasst, die in einem Gerüst dispergiert sind, das aus einem nicht-porösen biokompatiblen Hydrogel-Polymer konstruiert ist, wobei das Gerüst weiterhin basischen Fibroblasten-Wachstumsfaktor (bFGF) und ein adipogenes Mittel umfasst, das in der Lage ist, die Differenzierung der Stammzellen zu einer Adipozytenzelle zu stimulieren, wobei das Gerüst eine dreidimensionale Konfiguration aufweist, die Makrokanäle von einer zum Fördern des Einwachsens von vaskulärem Gewebe ausreichenden Größe umfasst.

2. Weichteilkonstrukt nach Anspruch 1, das weiter **dadurch gekennzeichnet ist, dass** es eine vorbestimmte dreidimensionale Gestalt und Dimension aufweist.

3. Weichteilkonstrukt nach Anspruch 1, wobei das Hydrogel-Polymer Polyethylenglycoldiacrylat, Polyethylenglycoldimethacrylat oder deren Gemische ist.

4. Weichteilkonstrukt nach Anspruch 3, wobei das Hydrogel-Polymer aus Polyethylenglycoldiacrylat besteht.

5. Weichteilkonstrukt nach Anspruch 1, das Fett- und Fasergewebe umfasst.

6. Weichteilkonstrukt nach Anspruch 1, wobei die adulten mesenchymalen Stammzellen aus Knochenmarkzellen stammen.

7. Weichteilkonstrukt nach Anspruch 1, wobei das Gerüst ein Material umfasst, das aus der Gruppe ausgewählt ist, die aus Polymilchsäure, Polyglycolsäure, einem Copolymer aus Polymilchsäure-Polyglycolsäure, Alginat, Chitosan, Agarose, Fibrin und Collagen besteht.

8. Weichteilkonstrukt nach Anspruch 1, wobei das adipogene Mittel aus der Gruppe ausgewählt ist, die aus Proglitazon, Dexamethason, Insulin, 3-Isobutyl-1-methylxanthin und L-Thyroxin besteht.

9. Verfahren zur Herstellung eines Weichteilkonstrukts, das eine vorbestimmte dreidimensionale Gestalt und Dimensionen aufweist, wobei das Verfahren umfasst:
(a) Bereitstellen von adulten mesenchymalen Stammzellen, die in einem Gerüst dispergiert sind, das aus einem nicht-porösen biokompatiblen Hydrogel-Polymer konstruiert ist, wobei das Gerüst vorbestimmte dreidimensionale Konfigurationen aufweist, die Makrokanäle von einer zum Fördern des Einwachsens von vaskulärem Gewebe ausreichenden Größe umfassen; und
(b) In-Kontakt-Bringen der adulten mesenchymalen Stammzellen mit einem adipogenen induzierenden Ergänzungsmittel und bFGF, das ausreicht, um die adulten mesenchymalen Stammzellen zu Adipozytenzellen und fibroblastengewebebildenden Zellen zu differenzieren;
wobei das Weichteilkonstrukt zum Implantieren in einen Wirt geeignet ist.

10. Verfahren nach Anspruch 9, wobei das Hydrogel-Polymer Polyethylenglycoldiacrylat ist.

11. Verfahren nach einem der Ansprüche 9 bis 10, wobei das Gerüst ein Material umfasst, das aus der Gruppe ausgewählt ist, die aus Polymilchsäure, Polyglycolsäure, einem Copolymer aus Polymilchsäure-Polyglycolsäure, Alginat, Chitosan, Agarose, Fibrin und Collagen besteht.

12. Weichteilkonstrukt nach einem der Ansprüche 1 bis 8 zur Verwendung in der Reparatur, Vergrößerung oder Rekonstruktion von Weichteilen eines Wirts.

13. Verwendung von Anspruch 12, wobei der Wirt ein Säuger ist.

## Revendications

1. Dispositif pour tissus mous comprenant des cellules souches mésenchymateuses adultes dispersées dans un échafaudage construit de polymère d'hydrogel biocompatible non poreux, dans lequel l'échafaudage comprend en outre un facteur de croissance des fibroblastes de base (bFGF) et un agent adipogénique apte à stimuler une différenciation des cellules souches dans des adipocytes, l'échafaudage présentant une configuration tridimensionnelle qui comprend des microcanaux d'une taille suffisante pour favoriser la croissance de tissus vasculaires.

2. Dispositif pour tissus mous selon la revendication l, **caractérisé en outre par le fait qu'**il présente une forme et une dimension tridimensionnelle prédéfinies.

3. Dispositif pour tissus mous selon la revendication 1, dans lequel le polymère d'hydrogel est du diacrylate de polyéthylène glycol, du diméthacrylate de polyéthylène glycol ou des mélanges de ceux-ci.

4. Dispositif pour tissus mous selon la revendication 3, dans lequel le polymère d'hydrogel est constitué de diacrylate de polyéthylène glycol.

5. Dispositif pour tissus mous selon la revendication 1, comprenant des tissus adipeux et fibreux.

6. Dispositif pour tissus mous selon la revendication 1, dans lequel les cellules souches mésenchymales adultes sont dérivées de cellules de la moelle osseuse.

7. Dispositif pour tissus mous selon la revendication 1, dans lequel l'échafaudage comprend un matériau sélectionné à partir du groupe constitué d'acide polylactique, d'acide polyglycolique, d'un copolymère d'acide polylactique - acide polyglycolique, d'alginate, de chitosane, d'agarose, de fibrine et de collagène.

8. Dispositif pour tissus mous selon la revendication 1, dans lequel l'agent adipogène est sélectionné parmi le groupe constitué de proglitazone, de dexaméthasone, d'insuline, de 3-isobutyle-1-méthylxanthine et de L-thyroxine.

9. Procédé de préparation d'un dispositif pour tissus mous présentant une forme et des dimensions tridimensionnelles prédéterminées, le procédé comprenant les étapes consistant à :
(a) fournir des cellules souches mésenchymateuses adultes dispersées dans un échafaudage construit de polymère d'hydrogel biocompatible non poreux, l'échafaudage ayant des configurations tridimensionnelle prédéterminées, comprenant des macrocanaux d'une taille suffisante pour favoriser la croissance de tissus vasculaires ; et
(b) mettre en contact les cellules souches mésenchymateuses adultes avec un supplément inducteur adipogène et un bFGF en quantité suffisante pour différencier les cellules souches mésenchymateuses adultes dans les adipocytes et les cellules de formation de tissus fibroblastiques ;
dans lequel le dispositif pour tissus mous est adapté à une implantation chez un hôte.

10. Procédé selon la revendication 9, dans lequel le polymère d'hydrogel est du diacrylate de polyéthylène glycol.

11. Procédé selon l'une quelconque des revendications 9 à 10, dans lequel l'échafaudage comprend un matériau sélectionné parmi le groupe constitué d'acide polylactique, d'acide polyglycolique, d'un copolymère d'acide polylactique - acide polyglycolique, de chitosane, d'agarose, de fibrine et de collagène.

12. Dispositif de tissus mous selon l'une quelconque des revendications 1 à 8 destiné à une utilisation dans la réparation, l'augmentation ou la reconstruction de tissus mous chez un hôte.

13. Utilisation selon la revendication 12, dans laquelle l'hôte est un mammifère.
